# EUROPÄISCHE PATENTSCHRIFT

(11) **EP 1 532 948 B1**
(45) Veröffentlichungstag und Bekanntmachung des Hinweises auf die Patenterteilung: **21.06.2006**
(21) Anmeldenummer: 03026582.1
(22) Anmeldetag: 18.11.2003
(51) Int. Cl.: A61F 2/44, A61F 2/46

(54) **Operationssystem zum Einsetzen von Bandscheibenimplantaten**
Surgical system for insertion of spinal disc prostheses
Système chirurgical pour l'insertion de disques intervertébraux prothétiques

(43) Veröffentlichungstag der Anmeldung: 25.05.2005
(73) Patentinhaber: Zimmer GmbH, 8404 Winterthur (CH)
(72) Erfinder: Filippi, Michael, 8200 Schaffhausen (CH); Casutt, Guido, 8544 Rickenbach (CH); Reinmuth, Jochen, 8406 Winterthur (CH); Heller, Mathias, 8352 Räterschen (CH); Etter, Christian, Dr. med, 4102 Binningen/BL (CH); Schwarzenbach, Othmar, Dr., 3612 Steffisburg (CH)
(74) Vertreter: Manitz, Finsterwald & Partner GbR

(56) Entgegenhaltungen:
- WO-A-01/01893
- US-A- 4 997 432
- US-A1- 2002 082 608
- US-A1- 2003 135 277

## Beschreibung

Die Erfindung betrifft ein Operationssystem zum Einsetzen von Bandscheibenimplantaten.

Bei derartigen Operationen besteht das Problem, dass vor allem die zum Aufspreizen der beiden benachbarten Wirbelkörper, zwischen die das Bandscheibenimplantat einzusetzen ist, verwendeten Instrumente - z.B. Distraktionszangen - nicht nur relativ viel Platz benötigen, wodurch der zur Verfügung stehende Arbeitsraum verkleinert wird, sondern darüber hinaus dem Operateur die Sicht auf das Operationsfeld nehmen.

Ein entsprechendes Operationssystem wird beispielsweise in WO 01/01893 beschrieben.

Aufgabe der Erfindung ist es, ein Operationssystem zum Einsetzen von Bandscheibenimplantaten zu schaffen, das bei möglichst einfachem Aufbau, einfacher Handhabbarkeit und zuverlässiger Funktionsweise den Operateur sowohl beim Vorbereiten des Bandscheibenfaches als auch beim Einsetzen des Bandscheibenimplantates so wenig wie möglich behindert.

Die Lösung dieser Aufgabe erfolgt durch die Merkmale des Anspruchs 1 und insbesondere dadurch, dass das Operationssystem wenigstens einen Satz von in ein Bandscheibenfach zwischen zwei benachbarten Wirbelkörpern einbringbaren Arbeitsplatten zur Vorbereitung der einander zugewandten Wirbelkörperflächen, wenigstens ein Bandscheibenimplantat, das zwei im implantierten Zustand an den vorbereiteten Wirbelkörperflächen anliegende Implantatplatten und einen zwischen die Implantatplatten einbringbaren Implantatkern umfasst, sowie eine Aufspreizeinrichtung zum Auseinanderdrücken der Arbeitsplatten und der Implantatplatten umfasst, wobei die Aufspreizeinrichtung wenigstens einen Distraktionsschuh mit zumindest einem durch Befüllen mit einem Fluid ausdehnbaren Ballon umfasst, der mit zumindest einem Adapterelement verbunden ist, das sowohl mit zumindest einer der Arbeitsplatten als auch mit wenigstens einer der Implantatplatten koppelbar ist.

Erfindungsgemäß wird mit dem Distraktionsschuh ein universelles Distraktionswerkzeug geschaffen, das aufgrund seiner Koppelungsmöglichkeit während der Benutzung zusammen mit der Arbeitsplatte bzw. Implantatplatte eine Einheit bildet und selbst nur wenig Platz benötigt. Es braucht lediglich die an den Ballon angeschlossene Zuführleitung für das Fluid aus dem Operationsfeld herausgeführt zu werden. Dies kann problemlos in einer Weise erfolgen, die weder den Arbeitsraum verkleinert noch eine Sichtbeeinträchtigung für den Operateur darstellt.

Durch die erfindungsgemäß vorgesehene Möglichkeit, den Distraktionsschuh sowohl an die Arbeitsplatte als auch an die Implantatplatte zu koppeln und somit die Aufspreizeinrichtung sowohl beim Vorbereiten des Bandscheibenfaches als auch beim Einsetzen des Bandscheibenimplantates zu verwenden, wird die Anzahl der erforderlichen Instrumente gering gehalten. Hinzu kommen die einer auf der Ballontechnik basierenden Distraktionsvorrichtung innewohnenden Vorteile, nämlich insbesondere der Wegfall einer komplizierten Mechanik, wie sie insbesondere bei bekannten Distraktionszangen vorhanden ist, die praktische Wartungsfreiheit aufgrund fehlender mechanischer Belastungen, die Möglichkeit zur Auslegung als Wegwerfartikel bei vertretbaren Kosten sowie die bei der Fluidzuleitung vorhandene Flexibilität hinsichtlich des Ortes der Krafterzeugung.

Ferner ist erfindungsgemäß von Vorteil, dass sich die Verteilung der Aufspreizkraft zwischen den Arbeitsplatten bzw. den Implantatplatten durch ein entsprechendes Ballondesign gezielt steuern lässt. Des Weiteren kann durch eine Anzeige des in der Aufspreizeinrichtung herrschenden Fluiddrucks ein vom Operateur während der Operation ablesbares Maß für die Distraktionshöhe bereitgestellt werden, wodurch reproduzierbare Distraktionsverhältnisse geschaffen werden können.

Weitere bevorzugte Ausführungsformen der Erfindung sind den abhängigen Ansprüchen, der Beschreibung sowie der Zeichnung zu entnehmen.

Der Distraktionsschuh kann mittels des Adapterelementes auf die Arbeitsplatte und die Implantatplatte aufschiebbbar sein, wobei vorzugsweise das Aufschieben im Wesentlichen senkrecht zur Aufspreizrichtung erfolgt.

Die Arbeitsplatte und die Implantatplatte können jeweils mit zumindest einer Aussparung zur Aufnahme des Adapterelementes versehen sein.

Um eine besonders einfache und sichere Handhabung zu ermöglichen, ist gemäß einer weiteren Ausführungsform vorgesehen, dass die Aussparungen zur Fixierung des Adapterelementes in Aufspreizrichtung jeweils mit einer Hinterschneidung versehen sind. Die Querschnittsform der Hinterschneidung kann grundsätzlich beliebig ausgestaltet sein. Beispielsweise ist die Hinterschneidung schwalbenschwanzförmig.

Um die Gefahr von Beschädigungen des Ballons vor und nach dem eigentlichen Aufspreizen zu reduzieren, ist vorzugsweise das Adapterelement mit einer Vertiefung zur Aufnahme der ungefüllten Ballonhülle versehen.

Des Weiteren ist der Ballon vorzugsweise mit dem Adapterelement fest verbunden. Die feste Verbindung wird beispielsweise durch Verkleben erzielt. Hierdurch stellt der Distraktionsschuh eine als Ganzes handhabbare Einheit dar.

Gemäß einem weiteren bevorzugten Ausführungsbeispiel wird vorgeschlagen, dass zumindest eine der Arbeitsplatten gabelförmig ausgebildet ist. Zur Vorbereitung des Bandscheibenfachs ist hierdurch die betreffende Wirbelkörperfläche über den zwischen den Gabelarmen der Arbeitsplatte vorhandenen Freiraum problemlos für Bearbeitungswerkzeuge zugänglich. Die Gabelarme selbst können als Distraktionsplattformen verwendet werden. Hierzu umfasst die Spreizeinrichtung vorzugsweise zwei Distraktionsschuhe, wobei jeder Gabelarm der Arbeitsplatte mit einem Distraktionsschuh gekoppelt werden kann.

Des Weiteren wird erfindungsgemäß vorgeschlagen, dass die Arbeitsplatten mittels wenigstens eines Führungselementes miteinander verbunden und in Aufspreizrichtung relativ zueinander längs des Führungselementes höhenverstellbar sind. Bevorzugt werden als Führungselemente mehrere Stifte oder Stäbe verwendet, welche z.B. in die untere (kaudale) Arbeitsplatte geschraubt werden und auf welche die obere (kraniale) Arbeitsplatte aufgesteckt wird.

Der Zugang zum Arbeitsraum zwischen den Wirbelkörpern bleibt unbeeinträchtigt, wenn gemäß einem weiteren Ausführungsbeispiel die Führungselemente in Aufschieberichtung jeweils hinter dem auf die Arbeitsplatte aufgeschobenen Distraktionsschuh verlaufen.

Eine weitere erfindungsgemäß vorgeschlagene Möglichkeit für eine nicht störende Positionierung der Führungselemente besteht darin, dass die Führungselemente jeweils bezüglich des Spreizschuhs seitlich nach außen versetzt verlaufen, insbesondere in einem Eckbereich der Arbeitsplatte.

Bevorzugt sind die Arbeitsplatten als Bearbeitungslehren ausgebildet und insbesondere mit Führungseinrichtungen für Bearbeitungswerkzeuge wie z.B. Finnenschläger und Domfräser versehen.

Gemäß einem weiteren bevorzugten Ausführungsbeispiel weist zumindest eine Arbeitsplatte an einem Ende eine um insbesondere 90° abgewinkelte Schürze auf. Hierdurch kann der Arbeitsplatte eine - von der Seite betrachtet - L-förmige Gestalt gegeben werden, weshalb die Arbeitsplatten auch als L-Platten bezeichnet werden. Die Schürze kann in vorteilhafter Weise als Anschlag beim Aufsetzen der Arbeitsplatte auf den jeweiligen Wirbelkörper dienen. Ferner können an der Schürze Führungseinrichtungen für Bearbeitungswerkzeuge ausgebildet werden, die beim Vorbereiten des Bandscheibenfaches zum Einsatz kommen.

Ferner ist erfindungsgemäß bevorzugt vorgesehen, dass die Implantatplatten jeweils auf ihrer Außenseite eine domförmige Erweiterung insbesondere in Form eines Kugelsegments aufweisen. Diese Dome sorgen für eine primäre Positionsstabilität des Implantats nach dem Einsetzen.

Des Weiteren wird erfindungsgemäß vorgeschlagen, dass die Außenseiten der Implantatplatten jeweils nach außen gewölbt sind. Diese Wölbungen sind bevorzugt zusätzlich zu den vorstehend erwähnten domförmigen Erweiterungen vorgesehen, und zwar derart, dass jeweils die Wölbung flacher ist, dafür jedoch in der Plattenebene eine größere Ausdehnung aufweist als der Dom.

Des Weiteren kann erfindungsgemäß vorgesehen sein, dass die Außenseiten der Implantatplatten jeweils einen sich zumindest über einen Teil des Umfangs der Implantatplatten erstreckenden ebenen Randbereich aufweisen.

Insgesamt lässt sich durch eine Ausgestaltung der Außenseiten der Implantatplatten jeweils mit vergleichsweise stark gekrümmter domförmiger Erweiterung, relativ flacher Wölbung und ebenem Randbereich ein konturoptimiertes Interface zum knöchernen Aufbau des Wirbelkörpers erzielen.

Ferner können die Implantatplatten jeweils auf ihrer Außenseite wenigstens einen insbesondere als Finne ausgebildeten Führungsvorsprung aufweisen. Hierdurch wird dem Implantat im eingesetzten Zustand Rotationsstabilität verliehen.

Des Weiteren wird erfindungsgemäß vorgeschlagen, dass die Implantatplatten jeweils auf ihrer Innenseite mit wenigstens einer Aussparung zur Aufnahme des Adapterelementes des Distraktionsschuhs versehen sind. Diese Aussparung kann dem insbesondere als Finne ausgebildeten Führungsvorsprung gegenüber liegen.

In einer besonders bevorzugten Ausführungsform ist vorgesehen, dass die Implantatplatten jeweils auf ihrer Innenseite eine Vertiefung zur Aufnahme des Implantatkerns aufweisen, wobei die zusammenwirkenden Artikulationsflächen der Vertiefung und des Implantatkerns jeweils Kugelteilflächen sind. Die Vertiefungen ermöglichen eine versenkte und damit herausrutschsichere Anordnung des Implantatkerns zwischen den Implantatplatten. Durch die Ausbildung der Artikulationsflächen als Teilflächen einer Kugel ist das erfindungsgemäße Bandscheibenimplantat hinsichtlich seiner Bewegungsmöglichkeiten rotationssymmetrisch.

Der Implantatkern weist vorzugsweise eine linsenartige Grundform auf. Insbesondere kann der Implantatkern zumindest näherungsweise die Form zweier mit ihren ebenen Seiten aufeinander liegender Kugelsegmente aufweisen, wobei jeweils der Kugelmittelpunkt des einen Kugelsegmentes innerhalb des anderen Kugelsegmentes liegt.

Um ein Herausrutschen des Implantatkerns aus dem durch die Vertiefungen oder Konkavitäten der Implantatplatten gebildeten Aufnahmeraum bei extremen Körperhaltungen sicher zu verhindern, kann vorgesehen sein, dass zumindest eine Implantatplatte einen von ihrer Innenseite abstehenden Zapfen aufweist, der bei zusammengesetztem Implantat in eine auf der Außenseite des Implantatkerns ausgebildete Ausnehmung hineinragt, wobei die Ausnehmung größer als der Zapfen bemessen ist, um eine Relativbewegung zwischen Implantatplatte und Implantatkern zu ermöglichen.

Der Zapfen und/oder das Zentrum des Implantatkerns können sowohl mittig als auch exzentrisch bezüglich der Abmessung der Implantatplatte in sagittaler Richtung angeordnet sein.

Um die benötigte Distraktionshöhe zum Einbringen des Implantatkerns zwischen die Implantatplatten möglichst gering zu halten, kann gemäß einem weiteren Ausführungsbeispiel vorgesehen sein, dass der Implantatkern auf zumindest einer Außenseite mit einem vom Rand zur Ausnehmung verlaufenden Einführkanal für den Zapfen der Implantatplatte versehen ist.

Eine alternative oder zusätzliche Möglichkeit, die Distraktionshöhe gering zu halten, besteht gemäß einer weiteren Ausführungsform darin, zumindest eine Implantatplatte auf ihrer Innenseite mit einem vom Rand zur Vertiefung verlaufenden Einführkanal für den Implantatkern zu versehen.

Die Erfindung wird im Folgenden beispielhaft unter Bezugnahme auf die Zeichnung beschrieben. Es zeigen:
- Fig. 1: eine kaudale Arbeitsplatte und zwei Distraktionsschuhe eines Operationssystems gemäß einer Ausführungsform der Erfindung,
- Fig. 2: die kaudale Arbeitsplatte von Fig. 1 sowie eine kraniale Arbeitsplatte,
- Fig. 3: die Arbeitsplatten von Fig. 2 im zusammengesetzten Zustand ohne Distraktionsschuhe und mit verschiedenen Bearbeitungswerkzeugen,
- Fig. 4a-4f: verschiedene Möglichkeiten für die Ausgestaltung einer kaudalen Arbeitsplatte eines erfindungsgemäßen Operationssystems,
- Fig. 5a: die Koppelung eines Distraktionsschuhs an eine Implantatplatte gemäß der Erfindung,
- Fig. 5b: eine schematische Querschnittsansicht eines erfindungsgemäßen Distraktionsschuhs,
- Fig. 6a-6e: verschiedene Ausgestaltungen eines erfindungsgemäßen Distraktionsschuhs,
- Fig. 7a-7g: verschiedene erfindungsgemäße Ballonkonfigurationen zusammen mit einer kaudalen Arbeitsplatte,
- Fig. 8a-8d: unterschiedliche Ansichten verschiedener erfindungsgemäßer Ballonkonfigurationen zusammen mit einer kaudalen Arbeitsplatte und einer kranialen Arbeitsplatte,
- Fig. 9: verschiedene Ansichten eines erfindungsgemäßen Bandscheibenimplantats,
- Fig. 10a+10b: verschiedene perspektivische Ansichten des Bandscheibenimplantats von Fig. 9,
- Fig. 11a-11c: jeweils eine Draufsicht auf eine gegenüber Fig. 9 modifizierte Ausführungsform eines Implantatkerns, und
- Fig. 12: eine perspektivische Ansicht eines gegenüber Fig. 9 modifizierten Bandscheibenimplantats.

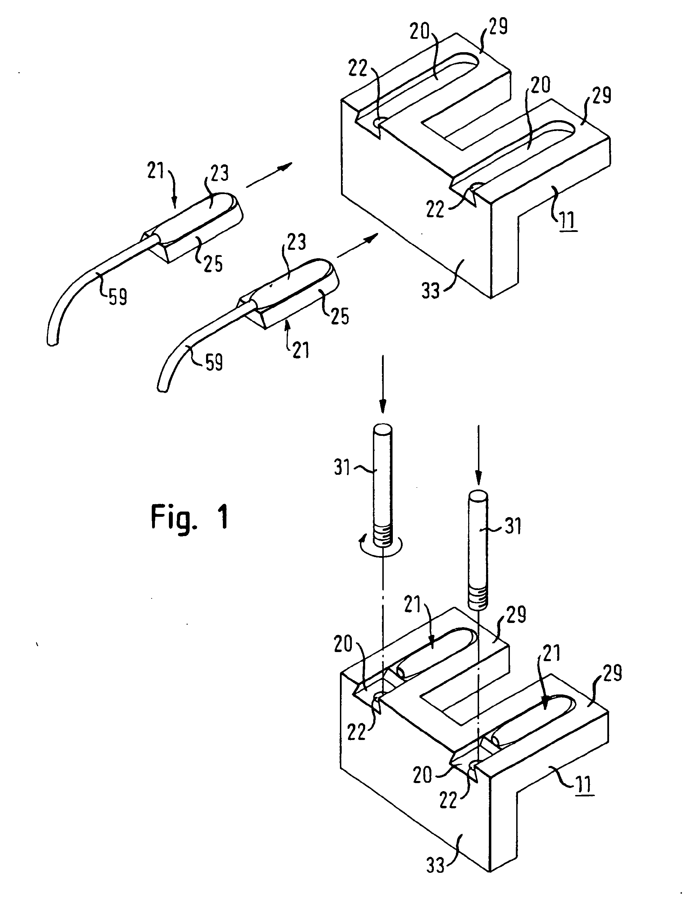

Das erfindungsgemäße Operationssystem umfasst zwei Arbeitsplatten 11, 13, ein Bandscheibenimplantat aus zwei Implantatplatten 15, 17 und einem Implantatkern 19 sowie eine Aufspreizeinrichtung mit zwei Distraktionsschuhen 21. Im Folgenden werden die einzelnen Bestandteile des Operationssystems anhand der Fig. 1-12 vorgestellt, woraufhin auf die Art und Weise der Verwendung des Operationssystems beim Einsetzen des Implantats eingegangen wird.

Fig. 1 zeigt eine gabelförmige kaudale Arbeitsplatte 11, die eine um 90° nach unten abgewinkelte Schürze 33 aufweist und somit - von der Seite betrachtet - L-förmig ausgebildet ist. Für die abgewinkelte Schürze 33 sind jedoch auch kleinere Winkel bis 75° möglich, wenn die späteren Implantatplatten in einem bestimmten Keilwinkel (Angulierung) zueinander stehen sollen. Auf der Oberseite der Gabelarme 29 der Arbeitsplatte 11 sind hinterschnittene nutförmige Aussparungen 20 ausgebildet, in die jeweils von der Seite der Schürze 33 her ein mit einer Fluidleitung verbundener Distraktionsschuh 21 einschiebbar ist, der ein Adapterelement 25 zur Koppelung mit der Aussparung 20 sowie einen an die Fluidleitung 59 angeschlossenen Ballon 23 umfasst, der durch Befüllen mit Fluid ausdehnbar ist, um auf diese Weise als Distraktionseinrichtung zu dienen.

Das untere Bild in Fig. 1 zeigt - der Einfachheit halber ohne Fluidleitungen 59 - die durch Aufschieben mit der Arbeitsplatte 11 gekoppelten Distraktionsschuhe 21 sowie zwei Führungsstifte 31, die nach dem Aufschieben der Distraktionsschuhe 21 mit den Gabelarmen 29 der Arbeitsplatte 11 verschraubt werden, und zwar über in den Koppelungsnuten 20 ausgebildete Gewindebohrungen 22.

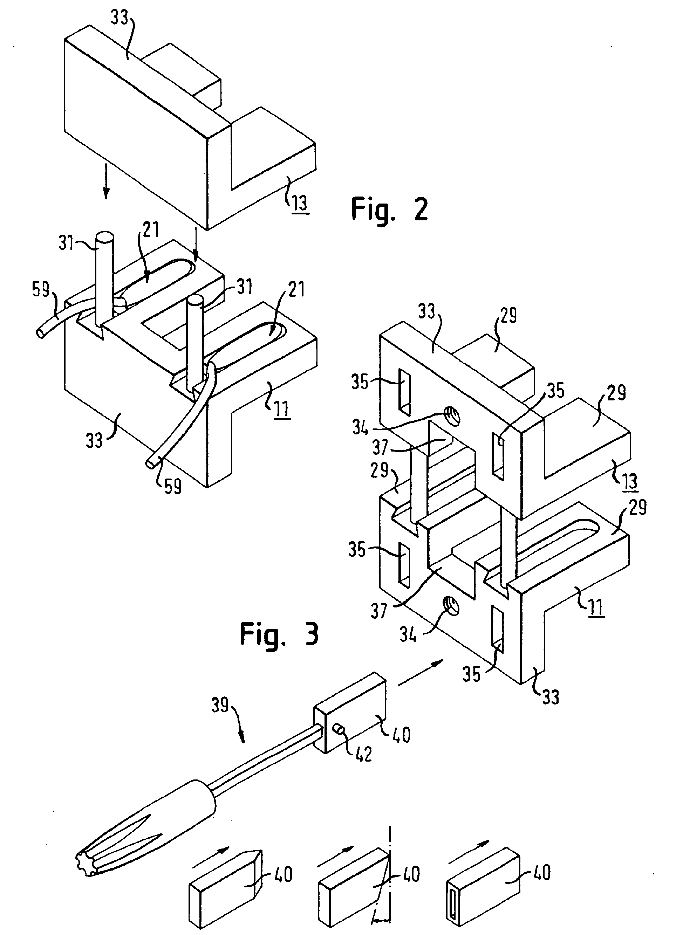

Fig. 2 zeigt zusätzlich zu der kaudalen Arbeitsplatte 11 mit den aufgeschobenen Distraktionsschuhen 21 und den in Aufschieberichtung hinter den Distraktionsschuhen 21 eingeschraubten, senkrecht zur Ebene der Arbeitsplatte 11 verlaufenden Führungsstiften 31 eine kraniale Arbeitsplatte 13, die ebenfalls gabelförmig ausgebildet ist und eine um 90° abgewinkelte Schürze 33 aufweist. Wie durch die Pfeile in Fig. 2 angedeutet, ist die obere Arbeitsplatte 13 auf die Führungsstifte 31 aufsteckbar, wodurch eine Zwangsführung realisiert wird, die lediglich eine Höhenverstellung der beiden Arbeitsplatten 11, 13 relativ zueinander längs der Führungsstifte 31 zulässt. Für diese Höhenverstellung sind die Distraktionsschuhe 21 vorgesehen.

Der Einfachheit halber nicht in Fig. 1 und 2, dafür jedoch in Fig. 3 dargestellt sind in den Schürzen 33 der Arbeitsplatten 11, 13 ausgebildete Durchgänge 35 rechteckigen Querschnitts, die als Führungseinrichtungen für einen so genannten Finnenschläger 39 dienen. Mit den Schlagelementen 40 des Finnenschlägers 39 werden in den Oberflächen der Wirbelkörper, an denen die Arbeitsplatten 11, 13 während der Vorbereitung des betreffenden Bandscheibenfachs anliegen, nut- oder rinnenförmige Vertiefungen für entsprechende Führungselemente des Implantates ausgebildet, worauf nachstehend näher eingegangen wird.

Wie Fig. 3 zeigt, können unterschiedlich ausgebildete Schlagelemente 40 zum Einsatz kommen. Die Schlagelemente 40 können mit einem als Tiefenbegrenzung dienenden Anschlagstift 42 versehen sein.

An den Schürzen 33 der Arbeitsplatten 11, 13 ausgebildete, der Einfachheit halber lediglich in Fig. 3 dargestellte Gewindebohrungen 34 dienen zur Anbringung von Instrumentenhaltern oder speziellen Zusatzeinrichtungen (nicht dargestellt), mit denen die Arbeitsplatten 11, 13 während der Operation gehalten und miteinander verriegelt werden können.

Wie Fig. 4a zeigt, ist eine rechteckige Form der Arbeitsplatten 11, 13 nicht zwingend. Die Arbeitsplatten 11, 13 können derart geformt sein, dass sie hinsichtlich Breite und Tiefe den gleichen Platzbedarf am Wirbelkörper 61 haben wie die betreffende Implantatplatte des einzusetzenden Bandscheibenimplantats.

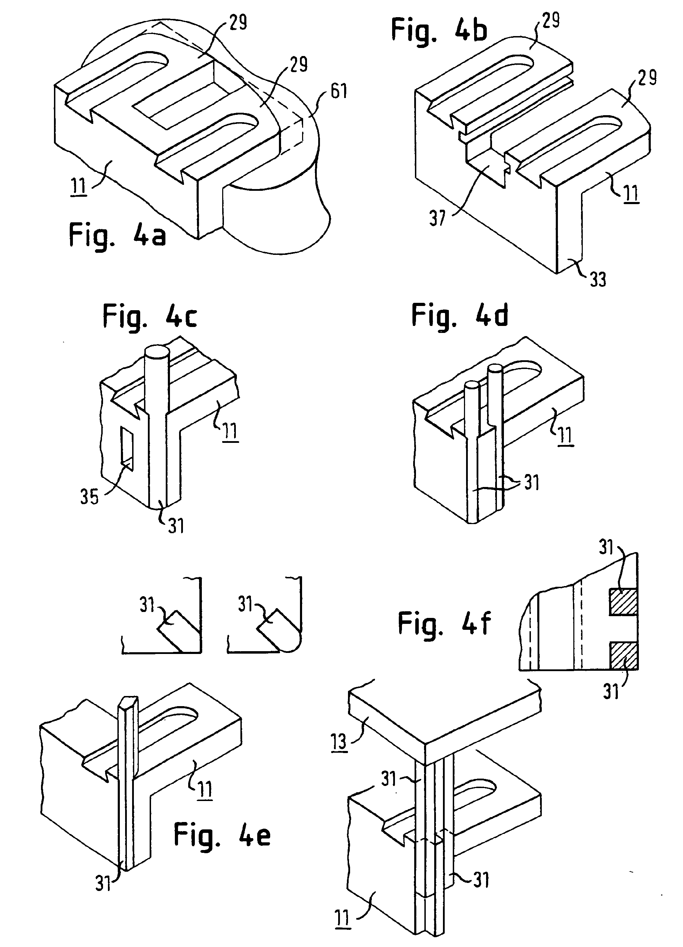

Fig. 4b zeigt weitere Führungseinrichtungen 37 für Bearbeitungswerkzeuge, die in diesem Beispiel zwischen den beiden Gabelarmen 29 der kaudalen Arbeitsplatte 11 vorgesehen sind. Die hier nicht dargestellte kraniale Arbeitsplatte 13 ist entsprechend ausgebildet (Fig. 3). Bei dieser Führungseinrichtung handelt es sich um einen oben offenen Einführkanal 37 mit T-förmigem Querschnitt in der Schürze 33 der Arbeitsplatte 11, wobei sich die den Querbalken des "T" bildenden Seitenkanäle des Einführkanals 37 weiter längs der Gabelarme 29 nach posterior erstrecken. Das jeweilige Bearbeitungswerkzeug, insbesondere ein so genannter Domfräser, kann so in einer definierten Relativlage bezüglich der Arbeitsplatte 11 und damit bezüglich des Wirbelkörpers 61 bewegt werden.

Die Fig. 4c-4f zeigen jeweils schematisch verschiedene Möglichkeiten zur Ausgestaltung der Führungselemente 31 der beiden Arbeitsplatten 11, 13. Anders als die in den Fig. 1, 2 und 3 gezeigten Führungselemente 31 verlaufen die stift- bzw. stabförmigen Führungselemente 31 hier jeweils in einem Eckbereich der Arbeitsplatten 11, 13.

Wie Fig. 4d und 4f zeigen, kann an den Eckbereichen jeweils ein Paar von Führungsstiften 31 vorgesehen sein. Den jeweils oben rechts in Fig. 4e und 4f gezeigten Querschnittsansichten ist zu entnehmen, dass die Führungselemente 31 unterschiedliche Querschnittsformen aufweisen können.

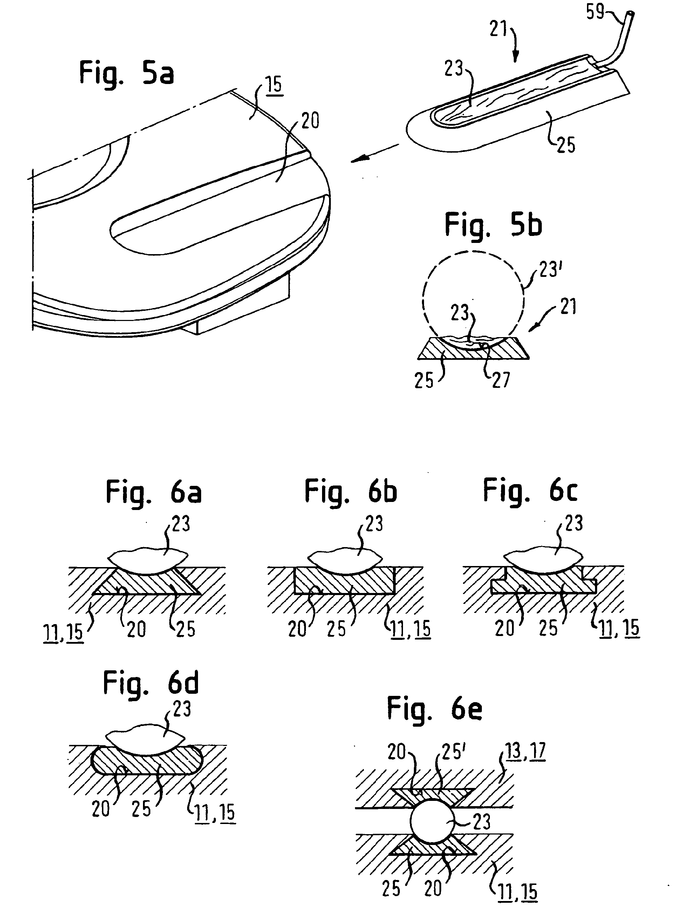

Fig. 5a zeigt teilweise eine Implantatplatte 15, die auf ihrer Innenseite mit einer hinterschnittenen Aussparung 20 für einen Distraktionsschuh 21 versehen ist. Auf das Bandscheibenimplantat selbst und damit auf die Implantatplatten wird nachstehend näher eingegangen. Die Aussparungen 20 in den Implantatplatten können zumindest hinsichtlich der Querschnittsform mit den Aussparungen 20 in den Gabelarmen 29 der Arbeitsplatten (Fig. 1) identisch sein, so dass die Distraktionsschuhe 21 sowohl zusammen mit den Arbeitsplatten als auch mit den Implantatplatten verwendet werden können.

Wie die Querschnittsdarstellung in Fig. 5b zeigt, ist das Adapterelement 25 des Distraktionsschuhs 21 auf seiner der ebenen Unterseite gegenüberliegenden Oberseite mit einer trog- oder wannenförmigen Vertiefung 27 versehen, in welcher die ungefüllte Ballonhülle 23 Platz findet, ohne zumindest wesentlich über den Rand der Vertiefung 27 hinauszuragen. Durch eine gestrichelte Linie in Fig. 5b ist die hier kreisförmige Querschnittsform des Ballons 23' im mit einem Fluid gefüllten Zustand angedeutet.

Die Fig. 6a-6d zeigen jeweils schematisch den Distraktionsschuh 21 im über das Adapterelement 25 mit einer Arbeitsplatte 11 oder einer Implantatplatte 15 gekoppelten Zustand. Vorzugsweise sind die Aussparungen 20 jeweils hinterschnitten (Fig. 6a, 6c und 6d), um die Distraktionsschuhe 21 bezüglich der Aufspreizrichtung sicher an der jeweiligen Platte 11, 15 zu fixieren. Zwingend ist dies jedoch nicht, d.h. es kann auch ohne eine derartige Fixierung gearbeitet werden, wie an dem Ausführungsbeispiel der Fig. 6b zu sehen ist.

Wie das Beispiel der Fig. 6e zeigt, ist es prinzipiell auch möglich, den Distraktionsschuh 21 gleichzeitig mit der unteren Platte 11, 15 und mit der oberen Platte 13, 17 zu koppeln. Hierzu ist der Ballon 23 mit zwei Adapterelementen 25, 25' verbunden.

Bei jeder der vorstehend erläuterten Ausführungsformen besteht eine bevorzugt durch Verkleben hergestellte feste Verbindung zwischen dem Ballon 23 und dem bzw. den Adapterelementen 25, 25'.

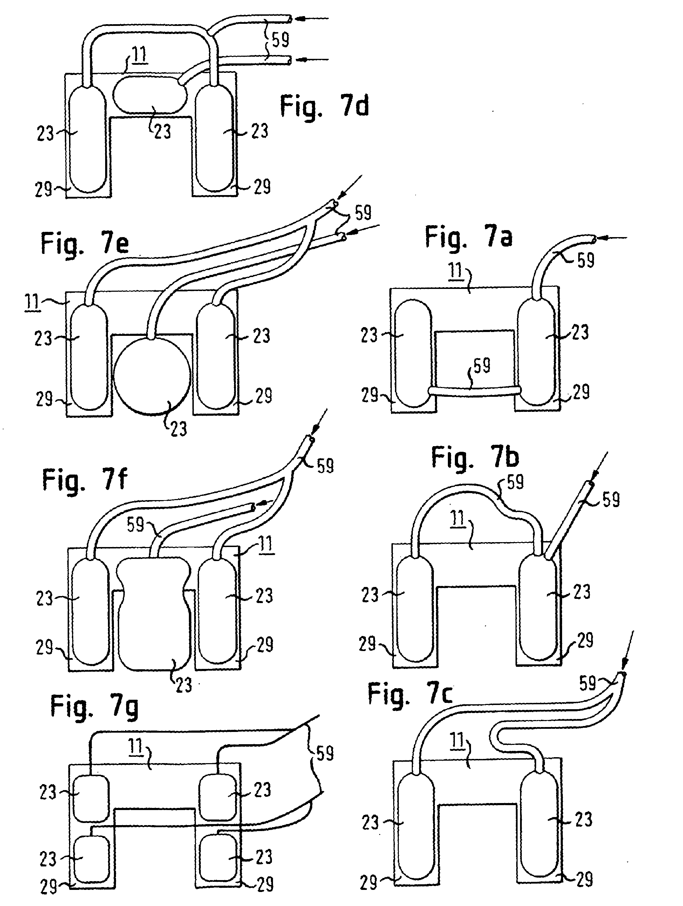

Die Fig. 7a-7c zeigen verschiedene Möglichkeiten für den Anschluss von zwei Ballons 23 an ein Fluidversorgungssystem, von dem hier lediglich die Fluidleitungen 59 dargestellt sind. Sowohl ein serieller (Fig. 7a und 7b) als auch ein - z.B. mittels eines Y-Stücks realisierbarer - paralleler (Fig. 7c) Anschluss ist möglich.

Während in den Ausführungsbeispielen der Fig. 7a-7c jeweils zwei Ballons 23 zum Einsatz kommen, die mit den Gabelarmen 29 der kaudalen Arbeitsplatte 11 gekoppelt werden und im Wesentlichen die gleiche Länge wie die Gabelarme 29 aufweisen, ist in den Ausführungsformen der Fig. 7d-7f jeweils ein zusätzlicher Ballon 23 außerhalb der Gabelarme 29 vorgesehen, während in dem Ausführungsbeispiel der Fig. 7g auf jedem Gabelarm 29 zwei Ballons 23 angeordnet sind, dafür aber kein zusätzlicher Ballon vorgesehen ist. Die zusätzlichen Ballons 23 der Beispiele in Fig. 7d-7f können aber auch bei dem Beispiel in Fig. 7g vorgesehen sein.

Der zusätzliche Ballon 23 kann auf dem die beiden Gabelarme 29 verbindenden Abschnitt, also auf der oberen Schmalseite der Schürze 33 (Fig. 1), der Arbeitsplatte 11 angeordnet werden (Fig. 7d), in dem Freiraum zwischen den beiden Gabelarmen 29 liegen (Fig. 7e) oder derart groß ausgeführt sein, dass er sowohl auf dem Verbindungsabschnitt aufliegt als auch in dem Freiraum zwischen den Gabelarmen 29 liegt.

In dem Ausführungsbeispiel der Fig. 7g sind ein anteriores Ballonpaar 23 und ein posteriores Ballonpaar 23 vorgesehen, so dass auf jedem Gabelarm 29 der Arbeitsplatte 11 zwei Ballons 23 hintereinander angeordnet sind. Jedem Ballonpaar 23 ist eine eigene Fluidleitung 59 zugeordnet.

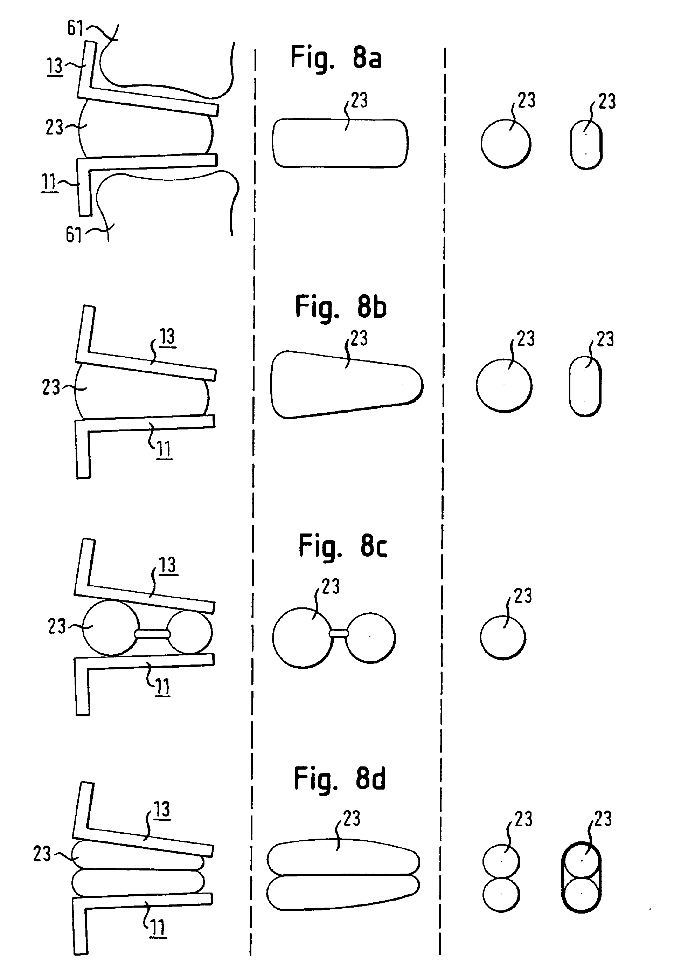

Die Fig. 8a-8d zeigen mögliche Ballonformen, und zwar jeweils in der linken Spalte unter Belastung im mit einem Fluid gefüllten Zustand beim Auseinanderdrücken der beiden Arbeitsplatten 11, 13 (in Fig. 8a sind zusätzlich die beiden Wirbelkörper 61 dargestellt, die in den Fig. 8b-8d der Einfachheit halber weg gelassen sind), in der mittleren Spalte in einer Seitenansicht die "natürliche" Form des gefüllten Ballons 23 ohne Belastung und in der rechten Spalte mögliche Querschnittsformen des gefüllten Ballons 23 ohne Belastung.

In dem Ausführungsbeispiel der Fig. 8a weist der Ballon 23 im gefüllten Zustand eine konstante Dicke auf, während der Ballon 23 in dem Ausführungsbeispiel der Fig. 8b im gefüllten Zustand keilförmig ausgebildet ist. Durch die Formgebung des Ballons 23 kann die Verteilung der Kraft, die beim Füllen des Ballons 23 von diesem auf die auseinander zu drückenden Arbeitsplatten 11, 13 übertragen wird, gezielt beeinflusst werden.

Wie Fig. 8c zeigt, können auch Ballons 23 mit einem Mehrkammersystem zum Einsatz kommen, bei dem die einzelnen Kammern über eine Engstelle miteinander verbunden sind, so dass lediglich ein einziger Anschluss an eine hier nicht dargestellte Fluidleitung erforderlich ist. Die Engstelle kann bereits bei der Herstellung des Ballons 23 ausgebildet werden. Alternativ zu einer derartigen "eingebauten" Engstelle ist es möglich, einen Ballon 23 nachträglich mittels einer Klammer oder Manschette mit einer Engstelle zu versehen und so als einen Mehrkammerballon auszubilden.

Der Ballon 23 in dem Ausführungsbeispiel der Fig. 8d weist ebenfalls mehrere Kammern auf, zwischen denen jedoch keine Fluidverbindung besteht, so dass für jede Kammer eine eigene Anschlussleitung (nicht gezeigt) zur Fluidversorgung vorgesehen ist. Wie die Querschnittsdarstellungen in der rechten Spalte der Fig. 8d zeigen, können die beiden Kammern gemäß der linken Darstellung aneinander befestigt sein, beispielsweise durch Verkleben, oder gemäß der rechten Darstellung durch eine gemeinsame Umhüllung zusammengehalten werden.

Als Material für die Ballonhülle kommt beispielsweise Nylon (Polyamid), Polyester (PET), Silikon, Latex oder Polyurethan (PU) in Frage. Die Oberfläche der Ballonhülle kann glatt, gerillt oder genoppt sein. Das Fluid zum Füllen der Ballons kann Wasser (z.B. eine sterile Ringerlösung) oder ein flüssiges Röntgenkontrastmittel (Lopamiro) sein. Der Ballon kann auf einen Fluiddruck von beispielsweise etwa 2 bis 10 bar gefüllt werden, wobei vorzugsweise eine manuell betätigbare Spritze zum Einbringen des Fluids in den Ballon vorgesehen ist. Eine motorische Unterstützung beim Befüllen durch eine Pumpe ist möglich. Die Spritze bzw. Pumpe kann mit einer Anzeigeeinrichtung zum Ablesen des Druckes versehen sein. Einer empirisch ermittelte Werte zur Abhängigkeit des Ballondurchmessers von dem herrschenden Fluiddruck enthaltenden Tabelle bzw. grafischen Darstellung kann während der Operation für den jeweils angezeigten Druck die momentane Distraktionshöhe, d.h. der Abstand zwischen den Arbeitsplatten 11, 13 bzw. den Implantatplatten 15, 17, entnommen werden.

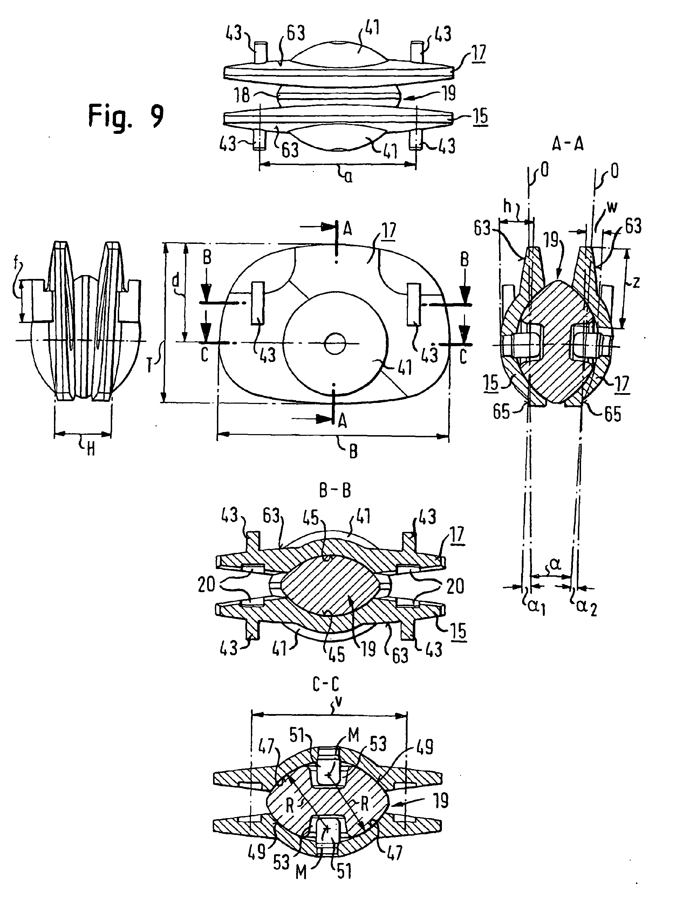

Fig. 9 zeigt verschiedene Ansichten des erfindungsgemäßen Bandscheibenimplantats, das zwei auch als Deckplatten oder Endplatten bezeichnete Implantatplatten 15, 17 sowie einen auch als Inlay bezeichneten Implantatkern 19 umfasst.

Der Implantatkern 19 besitzt eine linsenartige Grundform, die zwei mit ihren ebenen Seiten aneinander liegenden Kugelsegmenten entspricht. Die äußeren Artikulationsflächen 49 des Implantatkerns 19 sind somit Kugelteilflächen. Wie insbesondere der oberen Seitenansicht in Fig. 9 zu entnehmen ist, entspricht die Form des Implantatkerns 19 nicht exakt zwei aufeinander gesetzten Kugelsegmenten, sondern es befindet sich zwischen den ebenen Seiten der Kugelsegmente eine Zwischenscheibe 18 von relativ geringer Höhe und mit geradem Rand.

An seinen Polen ist der Implantatkern 19 mit Ausnehmungen 53 versehen, in die bei zusammengesetztem Implantat Zapfen 51 der Implantatplatten 15, 17 hineinragen, worauf nachstehend näher eingegangen wird.

Wie insbesondere den Schnitten B-B und C-C zu entnehmen ist, sind die Implantatplatten 15, 17 jeweils auf ihrer Außenseite mit einer relativ flachen Wölbung 63 versehen, auf der sich wiederum eine stärker gekrümmte domförmige Erweiterung 41 erhebt, die einer Vertiefung 45 auf der Innenseite der Implantatplatte 15, 17 entspricht, deren Artikulationsfläche 47 ebenfalls eine Teilfläche einer Kugel ist, deren Radius demjenigen der Artikulationsflächen 49 des Implantatkerns 19 entspricht. Wie insbesondere der Schnitt C-C zeigt, besteht im zusammengesetzten Zustand des Implantats vollflächiger Kontakt zwischen den beiden Artikulationsflächen 47, 49. Für jedes Kugelsegment des Implantatkerns 19 liegt der Mittelpunkt M der Kugel, auf deren Oberfläche die Artikulationsflächen 47, 49 liegen, innerhalb des jeweils anderen Kugelsegmentes, und zwar im Bereich der Ausnehmung 53.

Auf ihren Außenseiten sind die Implantatplatten 15, 17 des Weiteren mit Finnen 43 versehen. Mit diesen Führungsvorsprüngen 43 werden die Implantatplatten 15, 17 beim Einsetzen in das Bandscheibenfach in zuvor mittels eines Finnenschlägers 39 (Fig. 3) vorbereiteten nutförmigen Ausnehmungen auf den Oberflächen der Wirbelkörper geführt.

Den Finnen 43 gegenüberliegend sind auf den Innenseiten der Implantatplatten 15, 17 die Aussparungen 20 zur Aufnahme eines Adapterelementes 25 eines Distraktionsschuhs 21 ausgebildet (Fig. 5a).

Der Durchmesser der in Form von separaten Elementen vorgesehenen Zapfen 51 der Implantatplatten 15, 17 (Schnitt C-C in Fig. 9) ist kleiner als derjenige der im Implantatkern 19 ausgebildeten Ausnehmungen 53. Die auf diese Weise mit Spiel in die Ausnehmungen 53 hineinragenden Zapfen 51 verhindern ein Herausrutschen des Implantatkerns 19 aus dem von den beiden Vertiefungen 45 gebildeten Aufnahmeraum bei extremen Körperhaltungen.

Wie insbesondere der Schnitt A-A in Fig. 9 zeigt, verlaufen die flachen Wölbungen 63 auf den Außenseiten der Implantatplatten 15, 17 jeweils nicht über den gesamten Umfang bis zum Plattenrand. Über einen Teilumfang der Implantatplatten 15, 17 erstreckt sich ein ebener Randbereich 65.

Der Schnitt A-A zeigt außerdem, dass die so genannte Angulation der Implantatplatten 15, 17 jeweils bezüglich einer Nullreferenz 0 gemessen wird, bei der es sich um eine Ebene handelt, die senkrecht zu den gestrichelt eingezeichneten Mittelachsen der Zapfen 51 verläuft. Der resultierende Angulationswinkel α des zusammengesetzten Implantats bei einer bestimmten Relativstellung zwischen dem Implantatkern 19 und den beiden Implantatplatten 15, 17 wird bestimmt durch die Summe aus der kaudalen Angulation α1 und der kranialen Angulation α2.

Aus der Draufsicht und aus Schnitt A-A ist ersichtlich, dass das Zentrum von Dom 41 und Zapfen 51 exzentrisch längs der Mittellinie nach posterior verschoben ist.

Das erfindungsgemäße Bandscheibenimplantat weist bestimmte charakteristische Größen auf, die bei der Implantatherstellung zur Optimierung des Implantats und zur Anpassung an die jeweilige Patientenanatomie variiert werden können. Hierbei handelt es sich insbesondere um die folgenden Parameter, deren Definition jeweils den verschiedenen Ansichten der Fig. 9 entnommen werden kann:
- H: Höhe des Implantats
- B: Breite des Implantats
- T: Tiefe des Implantats
- R: Radius der Artikulationsflächen
- d: Domposition
- h: Domhöhe
- z: Wölbungszentrum
- w: Wölbungshöhe
- a: Finnenabstand
- f: Finnenhöhe
- v: Abstand der Aussparungen

Abweichend von dem in Fig. 9 dargestellten Ausführungsbeispiel können die Zapfen 51 auch weggelassen werden. Eine derartige alternative Ausgestaltung kommt insbesondere dann in Frage, wenn die Vertiefungen 45 in den Implantatplatten 15, 17 derart ausgebildet sind oder ausgebildet werden können, dass sie alleine bereits eine ausreichende Extrusionssicherheit bietet, d.h. mit ausreichender Sicherheit ein Herausrutschen des Implantatkerns 19 verhindern.

Die Implantatplatten 15, 17 können aus einer CoCr-Legierung oder einer Titan-Legierung hergestellt und auf der äußeren Knochenseite mit porösem Titan und gegebenenfalls zusätzlich mit Hydroxy-Apatit (HAC) beschichtet sein, um auf diese Weise ein besonders schnelles Anwachsen des Knochens zu ermöglichen. In der Praxis steht bevorzugt ein Satz von unterschiedlich großen Implantatplatten 15, 17 zur Verfügung, um eine optimale Anpassung an verschiedene Patientenanatomien zu erreichen. Die Implantatplatten 15, 17 können sich insbesondere hinsichtlich ihrer Breite, Tiefe und Winkelung (Angulation) voneinander unterscheiden.

Der Implantatkern 19 kann beispielsweise aus Polyethylen oder Metall bestehen. Dabei ist Polyethylen das bevorzugte Material, da hierdurch axial einwirkende Kräfte besser elastisch aufgenommen werden können, d.h. ein besseres axiales Dämpfungsvermögen vorhanden ist. Um einen eventuell möglichen Abrieb zu vermeiden, kann eine dünne Metallschale über das Kunststoffmaterial gelegt werden. Es entsteht dann eine Kombination von metallischen Kugelteilflächen, die sich aufgrund ihrer Kugelform in enorm hoher Präzision zueinander herstellen lassen. Ein derartiges Metall-Metall-Zusammenspiel ist allgemein in der europäischen Patentanmeldung 97903208.3 (Veröffentlichungsnummer EP 0 892 627) beschrieben, auf deren Inhalt hiermit zur Ergänzung der Offenbarung der vorliegenden Anmeldung ausdrücklich Bezug genommen wird.

Durch die versenkte Anordnung des Implantatkerns 19 in den Konkavitäten 45 der Implantatplatten 15, 17 wird eine relativ große Kraftübertragungsfläche bereitgestellt und damit eine vergleichsweise kleine Flächenlast erreicht, wobei gleichzeitig die Extrusionsgefahr gering gehalten wird.

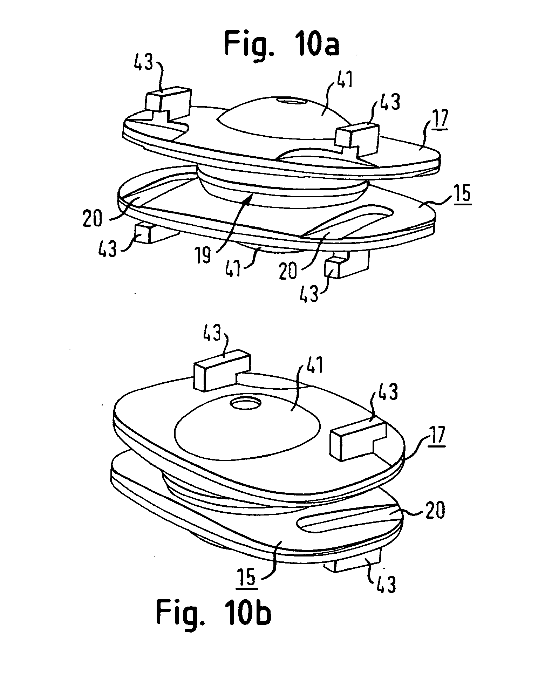

Die perspektivischen Darstellungen des Implantats in den Fig. 10a und 10b zeigen insbesondere die auf den Innenseiten der Implantatplatten 15, 17 ausgebildeten Aussparungen 20 für die Distraktionsschuhe (Fig. 5a) und die Ausgestaltung der Außenseiten der Implantatplatten 15, 17 mit dem Dom 41 und den Finnen 43.

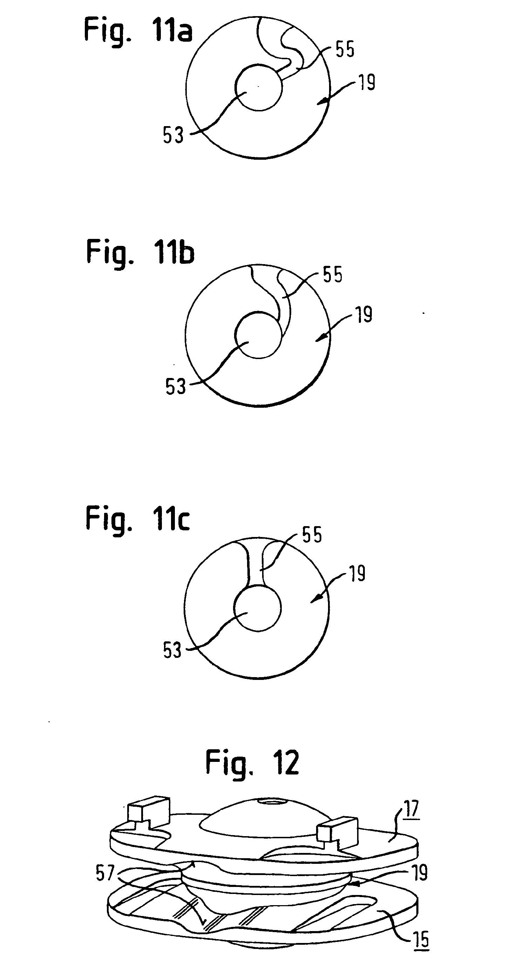

Die Fig. 11a- 11c sowie Fig. 12 zeigen mögliche Maßnahmen, die am Implantatkern 19 (Fig. 11a-11c) und an den Innenseiten der Implantatplatten 15, 17 (Fig. 12) getroffen werden können, um das Maß, um welches die Implantatplatten 15, 17 zum Einführen des Implantatkerns 19 auseinander gedrückt werden müssen, möglichst gering zu halten.

Gemäß Fig. 11a- 11c ist auf der Außenseite des Implantatkerns 19 jeweils ein sich vom Rand des Implantatkerns 19 bis zu der zentralen Ausnehmung 53 erstreckender Einführkanal 55 ausgebildet. Der Einführkanal 55 kann grundsätzlich einen beliebig gekrümmten Verlauf aufweisen und dabei entweder im Wesentlichen radial (Fig. 11a) oder tangential (Fig. 11 b) in die Ausnehmung 53 münden. Alternativ kann der Einführkanal 55 einen geradlinigen radialen Verlauf aufweisen (Fig. 11c).

Beim Einführen des Implantatkerns 19 zwischen die Implantatplatten 15, 17 ragen die Zapfen 51 in die Einführkanäle 55 des Implantatkerns 19 hinein, so dass die Zapfen 51 auch bei geringerem Plattenabstand dem einzuführenden Implantatkern 19 nicht im Wege sind.

Alternativ oder zusätzlich zu den Einführkanälen 55 des Implantatkerns 19 können die Implantatplatten 15, 17 jeweils auf ihrer Innenseite mit einem Einführkanal 57 in Form einer rinnenartigen Ausnehmung versehen sein, die von dem anterioren Plattenrand bis zur Vertiefung 45 verläuft, wodurch insgesamt ein sich von der anterioren Seite bis zum Aufnahmeraum für den Implantatkern 19 erstreckender "Einführtunnel" für den Implantatkern 19 vorhanden ist. Der Implantatkern 19 ist bereits zu Beginn des Einführvorgangs teilweise in die Einführkanäle 57 aufgenommen, so dass die Implantatplatten 15, 17 weniger weit auseinander gedrückt zu werden brauchen.

Bei einer Operation zum Einsetzen des erfindungsgemäßen Bandscheibenimplantats erfolgt die Vorbereitung des Bandscheibenfaches bis zum dem Zeitpunkt, in dem das erfindungsgemäße Operationssystems zum Einsatz kommt, wie bisher, d.h. das Räumen der natürlichen Zwischenwirbelscheibe erfolgt ohne das erfindungsgemäße Operationssystem. Auch eine erste Vorbereitung der Endplatten der Wirbelkörper, insbesondere mit einem so genannten "scharfen Löffel" (z.B. Cobb), erfolgt ohne Verwendung der erfindungsgemäßen Arbeitsplatten 11, 13.

Im Anschluss an diese erste Vorbereitung des Bandscheibenfaches wird das erfindungsgemäße Operationssystem beispielsweise wie folgt eingesetzt:

Zunächst werden die Ballons und eine Pumpe zum Befüllen der Ballons mit dem Fluid vorbereitet, indem eine Spritze mit einer sauberen Lösung gefüllt wird, die Ballons möglichst luftleer gepresst werden, die Spritze auf ein Y-Stück der Fluidzuleitung für die Ballons aufgesetzt wird und insbesondere bei hängenden Ballons durch mehrere aufeinander folgende Hübe der Spritze die Luft aus den Ballons gepumpt wird. Das Fluidsystem einschließlich der die Ballons umfassenden Distraktionsschuhe, der Fluidleitungen, des Fluidreservoirs und der Spritze, ggf. auch der Pumpe, kann teilweise oder komplett als gebrauchsfertig und steril verpackter Wegwerfartikel konzipiert sein.

Anschließend wird die Pumpe blasenfrei mit sauberer Lösung gefüllt, die Fluidzuleitung der Ballons mit der Pumpe verbunden und zunächst durch Druckbeaufschlagung eine Dichtigkeitsüberprüfung durchgeführt. Anschließend wird das System drucklos gemacht.

Als nächstes werden die jeweils aus Adapterelement 25 und Ballon 23 bestehenden Distraktionsschuhe 21 mit der kaudalen Arbeitsplatte 11 gekoppelt und die Führungsstifte 31 mit der kaudalen Arbeitsplatte 11 verschraubt (Fig. 1). Die Fluidleitungen 59 werden hierbei lateral außen an den Führungsstiften 31 vorbei nach anterior weggeführt (Fig. 2).

Anschließend wird die kraniale Arbeitsplatte 13 auf die Führungsstifte 31 aufgesteckt (Fig. 3). Es erfolgt zunächst eine Funktionsüberprüfung durch eine kurzzeitige Druckbeaufschlagung. Anschließend wird ein Instrumentenhalter mit der kaudalen Arbeitsplatte 11 verschraubt, woraufhin die kraniale Arbeitsplatte 13 mittels des Instrumentenhalters oder eines speziellen Aufsatzes des Instrumentenhalters in einer vorgegebenen minimalen Höhe relativ zur kaudalen Arbeitsplatte 11 gesichert bzw. verriegelt wird.

Daraufhin wird die auf diese Weise vorbereitete Aufspreizeinrichtung in das Bandscheibenfach eingeführt. Nach Entriegelung der kranialen Arbeitsplatte 13 wird mittels der Pumpe der Druck zunächst auf etwa 1 bar erhöht, woraufhin eine erste Lagekontrolle durchgeführt wird. Anschließend wird schrittweise der Druck auf etwa 10 bar erhöht, wobei während der hierdurch erfolgenden Ausdehnung der zunehmend mit dem Fluid gefüllten Ballons 23 und der daraus resultierenden Aufspreizung der Arbeitsplatten 11, 13 und damit der Wirbelkörper 61 immer wieder Lagekontrollen durchgeführt werden.

Der dabei noch mit der kaudalen Arbeitsplatte 11 verbundene Instrumentenhalter wird anschließend entfernt. Nun kann die eigentliche Vorbereitung der Wirbelkörperflächen erfolgen, indem mittels eines Domfräsers die Vertiefungen für die Dome 41 der Implantatplatten 15, 17 gefräst und mittels des Finnenschlägers 39 (Fig. 3) die Aussparungen für die Finnen 43 der Implantatplatten 15, 17 hergestellt werden. Für diese Bearbeitungswerkzeuge sind die in Verbindung mit Fig. 3 und Fig. 4b beschriebenen, an den Arbeitsplatten 11, 13 ausgebildeten Führungseinrichtungen 35, 37 vorgesehen. Die Flächen der Wirbelkörper sind über den Freiraum zwischen den Gabelarmen 29 der Arbeitsplatten 11, 13 für den Domfräser zugänglich, der über die auch als Instrumentenkanal bezeichnete Führungseinrichtung 37 zwischen den beiden Gabelarmen 29 zugeführt werden kann.

Anschließend wird nach erneutem Einschrauben des Instrumentenhalters der Druck reduziert und Fluid aus dem System abgelassen, die Spreizeinrichtung entfernt und demontiert, wobei die Distraktionsschuhe 21 weiter bereitgehalten werden, da sie erneut verwendet werden, nämlich in Verbindung mit den Implantatplatten 15, 17, in deren Aussparungen 20 die Distraktionsschuhe 21 nach dem Abnehmen von den Arbeitsplatten 11, 13 eingeschoben werden (Fig. 5a).

Im Anschluss an die Montage des Implantatkerns 19 an einen Halter, die Montage der Implantatplatten 15, 17 an eine Einführzange und das vorstehend bereits erwähnte Aufschieben der Distraktionsschuhe 21 auf die Implantatplatten 15, 17 wird zunächst bei auf Minimalabstand eingestellter Einführzange ein geringer Druck auf die Ballons 23 gegeben, um auf diese Weise für eine Verrutschsicherung zu sorgen.

Anschließend wird mittels der Einführzange die Anordnung aus Implantatplatten 15, 17 mit dazwischen befindlichen Distraktionsschuhen 21 in das vorbereitete Bandscheibenfach eingeführt. Im Anschluss an eine erste Lagekontrolle wird das System unter Druck gesetzt und schrittweise unter Vornahme mehrerer Zwischen-Lagekontrollen der Druck weiter erhöht, wodurch die Implantatplatten 15, 17 und damit die Wirbelkörper 61 von den sich zunehmend ausdehnenden Ballons 23 auseinander gedrückt werden.

Sobald die angestrebte Distraktionshöhe erreicht ist, wird der Implantatkern 19 in den von den Konkavitäten 45 gebildeten Aufnahmeraum zwischen den Implantatplatten 15, 17 eingeführt. Daraufhin wird der Druck reduziert, ein Teil des Fluids aus den Ballons 23 abgelassen und der Sitz des Implantats durch Bewegen des Implantatkerns 19 mittels des Halters überprüft, welcher dann entfernt wird.

Die primäre Positionsstabilität des Implantats wird durch die Dome 41 der Implantatplatten 15, 17 erreicht, wohingegen die Finnen 43 der Implantatplatten 15, 17 für die primäre Rotationsstabilität sorgen. Die Höhe der Finnen 43 ist derart gewählt, dass auch bei vergleichsweise stark konkaver Ausbildung der Wirbelkörperflächen zumindest ein Teil der Finnen 43 stets mit dem Knochen in Eingriff steht. Dies ist insbesondere deshalb von Bedeutung, da im Allgemeinen die erwähnte Konkavität der Wirbelkörper mit ihrem Alter zunimmt.

### Bezugszeichenliste

- 11: kaudale Arbeitsplatte
- 13: kraniale Arbeitsplatte
- 15: Implantatplatte
- 17: Implantatplatte
- 18: Zwischenscheibe
- 19: Implantatkern
- 20: Aussparung für Adapterelement
- 21: Distraktionsschuh
- 22: Gewindebohrung
- 23, 23': Ballon
- 25, 25': Adapterelement
- 27: Vertiefung des Adapterelementes
- 29: Gabelarm der Arbeitsplatte
- 31: Führungselement
- 33: Schürze
- 34: Gewindebohrung
- 35: Führungseinrichtung
- 37: Führungseinrichtung
- 39: Bearbeitungswerkzeug
- 40: Schlagelement
- 41: domförmige Erweiterung
- 42: Anschlagstift
- 43: Führungsvorsprung, Finne
- 45: Vertiefung der Implantatplatte
- 47: Artikulationsfläche der Vertiefung
- 49: Artikulationsfläche des Implantatkerns
- 51: Zapfen
- 53: Ausnehmung
- 55: Einführkanal des Implantatkerns
- 57: Einführkanal der Implantatplatte
- 59: Fluidleitung
- 61: Wirbelkörper
- 63: Wölbung
- 65: Randbereich

- M: Kugelmittelpunkt
- R: Radius der Artikulationsflächen
- 0: Nullreferenz
- α: Angulation

- H: Höhe der Implantatplatten
- B: Breite der Implantatplatten
- T: Tiefe der Implantatplatten
- d: Domposition
- h: Domhöhe
- z: Wölbungszentrum
- w: Wölbungshöhe
- a: Finnenabstand
- f: Finnenhöhe
- v: Abstand der Aussparungen

## Patentansprüche

1. Operationssystem zum Einsetzen von Bandscheibenimplantaten mit
- wenigstens einem Satz von in ein Bandscheibenfach zwischen zwei benachbarten Wirbelkörpern einbringbaren Arbeitsplatten (11, 13) zur Vorbereitung der einander zugewandten Wirbelkörperflächen,
- wenigstens einem Bandscheibenimplantat, das zwei im implantierten Zustand an den vorbereiteten Wirbelkörperflächen anliegende Implantatplatten (15, 17) und einen zwischen die Implantatplatten (15, 17) einbringbaren Implantatkern (19) umfasst, und
- einer Aufspreizeinrichtung zum Auseinanderdrücken der Arbeitsplatten (11, 13) und der Implantatplatten (15, 17),
wobei die Aufspreizeinrichtung wenigstens einen Distraktionsschuh (21) mit zumindest einem durch Befüllen mit einem Fluid ausdehnbaren Ballon (23) umfasst, der mit zumindest einem Adapterelement (25) verbunden ist, das sowohl mit zumindest einer der Arbeitsplatten (11, 13) als auch mit wenigstens einer der Implantatplatten (15, 17) koppelbar ist.

2. Operationssystem nach Anspruch 1,
**dadurch gekennzeichnet,**
**dass** der Distraktionsschuh (21) mittels des Adapterelementes (25) auf die Arbeitsplatte (11, 13) und die Implantatplatte (17, 19) aufschiebbar ist, bevorzugt im Wesentlichen senkrecht zur Aufspreizrichtung.

3. Operationssystem nach Anspruch 1 oder 2,
**dadurch gekennzeichnet,**
**dass** die Arbeitsplatte (11, 13) und die Implantatplatte (15, 17) jeweils mit zumindest einer Aussparung (20) zur Aufnahme des Adapterelementes (25) versehen sind.

4. Operationssystem nach Anspruch 3,
**dadurch gekennzeichnet,**
**dass** die Aussparungen (20) zur Fixierung des Adapterelementes (25) in Aufspreizrichtung jeweils mit einer insbesondere schwalbenschwanzförmigen Hinterschneidung versehen sind.

5. Operationssystem nach einem der vorhergehenden Ansprüche,
**dadurch gekennzeichnet,**
**dass** das Adapterelement (25) mit einer Vertiefung (27) zur Aufnahme der ungefüllten Ballonhülle (23) versehen ist.

6. Operationssystem nach einem der vorhergehenden Ansprüche,
**dadurch gekennzeichnet,**
**dass** der Ballon (23) mit dem Adapterelement (25) fest verbunden ist, insbesondere durch Verkleben.

7. Operationssystem nach einem der vorhergehenden Ansprüche,
**dadurch gekennzeichnet,**
**dass** zumindest eine der Arbeitsplatten (11, 13) gabelförmig ausgebildet ist.

8. Operationssystem nach Anspruch 7,
**dadurch gekennzeichnet,**
**dass** die Spreizeinrichtung wenigstens zwei Distraktionsschuhe (21) umfasst, wobei jeder Gabelarm (29) der Arbeitsplatte (11) mit einem Distraktionsschuh (21) koppelbar ist.

9. Operationssystem nach einem der vorhergehenden Ansprüche,
**dadurch gekennzeichnet,**
**dass** die Arbeitsplatten (11, 13) mittels wenigstens eines insbesondere stiftförmigen Führungselementes (31) miteinander verbindbar und in Aufspreizrichtung relativ zueinander längs des Führungselementes (31) höhenverstellbar sind.

10. Operationssystem nach Anspruch 9,
**dadurch gekennzeichnet,**
**dass** zumindest ein Führungselement (31) in Aufschieberichtung hinter dem auf die Arbeitsplatte (11) aufgeschobenen Distraktionsschuh (21) verläuft.

11. Operationssystem nach Anspruch 9 oder 10,
**dadurch gekennzeichnet,**
**dass** zumindest ein Führungselement (31) bezüglich des Distraktionsschuhs (21) seitlich nach außen versetzt verläuft, insbesondere in einem Eckbereich der Arbeitsplatte (11, 13).

12. Operationssystem nach einem der vorhergehenden Ansprüche,
**dadurch gekennzeichnet,**
**dass** zumindest eine Arbeitsplatte (11, 13) an einem Ende eine um insbesondere 90° abgewinkelte Schürze (33) aufweist.

13. Operationssystem nach einem der vorhergehenden Ansprüche,
**dadurch gekennzeichnet,**
**dass** die Arbeitsplatten (11, 13) als Bearbeitungslehren ausgebildet und insbesondere mit Führungseinrichtungen (35, 37) für Bearbeitungswerkzeuge (39), bevorzugt für Finnenschläger und Domfräser, versehen sind.

14. Operationssystem nach einem der vorhergehenden Ansprüche,
**dadurch gekennzeichnet,**
**dass** die Implantatplatten (15, 17) jeweils auf ihrer Außenseite eine domförmige Erweiterung (41) insbesondere in Form eines Kugelsegmentes aufweisen.

15. Operationssystem nach einem der vorhergehenden Ansprüche,
**dadurch gekennzeichnet,**
**dass** die Außenseiten der Implantatplatten (15, 17) jeweils nach außen gewölbt sind, wobei vorzugsweise die Wölbungen (63) zusätzlich zu domförmigen Erweiterungen (41) vorgesehen sind.

16. Operationssystem nach einem der vorhergehenden Ansprüche,
**dadurch gekennzeichnet,**
**dass** die Außenseiten der Implantatplatten (15, 17) jeweils einen sich zumindest über einen Teil des Umfangs der Implantatplatten (15, 17) erstreckenden ebenen Randbereich (65) aufweisen.

17. Operationssystem nach einem der vorhergehenden Ansprüche,
**dadurch gekennzeichnet,**
**dass** die Implantatplatten (15, 17) jeweils auf ihrer Außenseite wenigstens einen insbesondere als Finne ausgebildeten Führungsvorsprung (43) aufweisen.

18. Operationssystem nach einem der vorhergehenden Ansprüche,
**dadurch gekennzeichnet,**
**dass** die Implantatplatten (15, 17) jeweils auf ihrer Innenseite mit wenigstens einer Aussparung (20) zur Aufnahme des Adapterelementes (25) des Distraktionsschuhs (21) versehen sind.

19. Operationssystem nach einem der vorhergehenden Ansprüche,
**dadurch gekennzeichnet,**
**dass** die Aussparung (20) einem insbesondere als Finne ausgebildeten Führungsvorsprung (43) gegenüber liegt.

20. Operationssystem nach einem der vorhergehenden Ansprüche,
**dadurch gekennzeichnet,**
**dass** die Implantatplatten (15, 17) jeweils auf ihrer Innenseite eine Vertiefung (45) zur Aufnahme des Implantatkerns (19) aufweisen, wobei vorzugsweise die zusammenwirkenden Artikulationsflächen (47, 49) der Vertiefung (45) und des Implantatkerns (19) jeweils Kugelteilflächen sind.

21. Operationssystem nach einem der vorhergehenden Ansprüche,
**dadurch gekennzeichnet,**
**dass** der Implantatkern (19) eine linsenartige Grundform aufweist.

22. Operationssystem nach einem der vorhergehenden Ansprüche,
**dadurch gekennzeichnet,**
**dass** der Implantatkern (19) zumindest näherungsweise die Form zweier mit ihren ebenen Seiten aufeinander liegenden Kugelsegmenten aufweist, wobei jeweils der Kugelmittelpunkt (11) des einen Kugelsegmentes innerhalb des anderen Kugelsegmentes liegt.

23. Operationssystem nach einem der vorhergehenden Ansprüche,
**dadurch gekennzeichnet,**
**dass** zumindest eine Implantatplatte (15, 17) einen von ihrer Innenseite abstehenden Zapfen (51) aufweist, der bei zusammengesetztem Implantat in eine auf der Außenseite des Implantatkerns (19) ausgebildete Ausnehmung (53) hineinragt, wobei die Ausnehmung (53) größer als der Zapfen (51) bemessen ist, um eine Relativbewegung zwischen Implantatplatte (15, 17) und Implantatkern (19) zu ermöglichen.

24. Operationssystem nach Anspruch 23,
**dadurch gekennzeichnet,**
**dass** der Zapfen (51) mittig bezüglich der Abmessung der Implantatplatte in sagittaler Richtung angeordnet ist.

25. Operationssystem nach Anspruch 23,
**dadurch gekennzeichnet,**
**dass** der Zapfen (51) exzentrisch bezüglich der Abmessung der Implantatplatte in sagittaler Richtung angeordnet ist.

26. Operationssystem nach einem der Ansprüche 23-25,
**dadurch gekennzeichnet,**
**dass** der Implantatkern (19) auf zumindest einer Außenseite mit einem vom Rand zur Ausnehmung (53) verlaufenden Einführkanal (55) für den Zapfen (51) der Implantatplatte (15, 17) versehen ist.

27. Operationssystem nach einem der vorhergehenden Ansprüche,
**dadurch gekennzeichnet,**
**dass** zumindest eine Implantatplatte (15, 17) auf ihrer Innenseite mit einem vom Rand zur Vertiefung (45) verlaufenden Einführkanal (57) für den Implantatkern (19) versehen ist.

## Claims

1. An operation system for the insertion of intervertebral disk implants comprising
- at least one set of work plates (11, 13) which can be introduced into a disk space between two adjacent vertebral bodies for the preparation of the vertebral body surfaces facing one another;
- at least one intervertebral disk implant which includes two implant plates (15, 17) contacting the prepared vertebral body surfaces in the implanted state and one implant core (19) which can be introduced between the implant plates (15, 17); and
- a spreading device to press apart the work plates (11, 13) and the implant plates (15, 17),
wherein the spreading device includes at least one traction shoe (21) with at least one balloon (23) which is expandable by being filled with a fluid and which is connected to at least one adapter element (25) which can be coupled both to at least one of the work plates (11, 13) and to at least one of the implant plates (15, 17).

2. An operation system in accordance with claim 1, **characterized in that** the traction shoe (21) can be pushed onto the work plate (11, 13) and onto the implant plate (17, 19) by means of the adapter element (25), preferably substantially perpendicular to the spreading direction.

3. An operation system in accordance with claim 1 or claim 2, **characterized in that**, the work plate (11, 13) and the implant plate (15, 17) are each provided with at least one cut-out (20) for the mounting of the adapter element (25).

4. An operation system in accordance with claim 3, **characterized in that** the cut-outs (20) are each provided with an undercut, in particular of dovetail shape, for the fixing of the adapter element (25) in the spreading direction.

5. An operation system in accordance with any one of the preceding claims, **characterized in that** the adapter element (25) is provided with a recess (27) for the mounting of the unfilled balloon skin (23).

6. An operation system in accordance with any one of the preceding claims, **characterized in that** the balloon (23) is firmly connected to the adapter element (25), in particular by adhesive bonding.

7. An operation system in accordance with any one of the preceding claims, **characterized in that** at least one of the work plates (11, 13) is made in fork shape.

8. An operation system in accordance with claim 7, **characterized in that** the spreading device includes at least two traction shoes (21), with each fork arm (29) of the work plate (11) being able to be coupled to a traction shoe (21).

9. An operation system in accordance with any one of the preceding claims, **characterized in that** the work plates (11, 13) can be connected to one another by means of at least one guide element (31), in particular of pin shape, and are vertically adjustable along the guide element (31) in the spreading direction relative to one another.

10. An operation system in accordance with claim 9, **characterized in that** at least one guide element (31) extends in the pushing-on direction behind the traction shoe (21) pushed onto the work plate (11).

11. An operation system in accordance with claim 9 or claim 10, **characterized in that** at least one guide element (31) extends laterally outwardly offset with respect to the traction shoe (21), in particular in a corner region of the work plate (11, 13) .

12. An operation system in accordance with any one of the preceding claims, **characterized in that** at least one work plate (11, 13) has an angled apron (33) at one end, in particular bent by 90°.

13. An operation system in accordance with any one of the preceding claims, **characterized in that** the work plates (11, 13) are preferably made as working templates and are in particular provided with guide arrangements (35, 37) for working tools (39), preferably for ball-peen hammers and dome cutters.

14. An operation system in accordance with any one of the preceding claims, **characterized in that** the implant plates (15, 17) each have a dome-shaped broadened portion (41), in particular in the form of a spherical segment, at their outer side.

15. An operation system in accordance with any one of the preceding claims, **characterized in that** the outer sides of the implant plates (15, 17) are each outwardly arched, with the arches (63) preferably being provided in addition to dome-shaped broadened portions (41).

16. An operation system in accordance with any one of the preceding claims, **characterized in that** the outer sides of the implant plates (15, 17) each have a planar marginal region (65) extending at least over part of the periphery of the implant plates (15, 17).

17. An operation system in accordance with any one of the preceding claims, **characterized in that** the implant plates (15, 17) each have at least one guide projection (43), in particular formed as a fin, on their outer side.

18. An operation system in accordance with any one of the preceding claims, **characterized in that** the implant plates (15, 17) are each provided on their inner sides with at least one cut-out (20) for the mounting of the adapter element (25) of the traction shoe (21).

19. An operation system in accordance with any one of the preceding claims, **characterized in that** the cut-out (20) lies opposite the guide projection (43) formed in particular as a fin.

20. An operation system in accordance with any one of the preceding claims, **characterized in that** the implant plates (15, 17) each have a recess (45) at their inner sides for the mounting of the implant core (19), with the cooperating articulation surfaces (47, 49) of the recess (45) and of the implant core (19) preferably each being partly spherical surfaces.

21. An operation system in accordance with any one of the preceding claims, **characterized in that** the implant core (19) has a lens-like basic shape.

22. An operation system in accordance with any one of the preceding claims, **characterized in that** the implant core (19) has at least approximately the shape of two spherical segments whose planar sides lie on top of one another, with the respective spherical center (11) of the one spherical segment lying within the other spherical segment.

23. An operation system in accordance with any one of the preceding claims, **characterized in that** at least one implant plate (15, 17) has a spigot (51) which protrudes from its inner side and which projects into a cut-out (53) formed on the outer side of the implant core (19), when the implant is assembled, with the cut-out (53) being dimensioned larger than the spigot (51) in order to permit a relative movement between the implant plate (15, 17) and the implant core (19).

24. An operation system in accordance with claim 23, **characterized in that** the spigot (51) is arranged centrally with respect to the dimension of the implant plate in the sagittal direction.

25. An operation system in accordance with claim 23, **characterized in that** the spigot (51) is arranged eccentrically with respect to the dimension of the implant plate in the sagittal direction.

26. An operation system in accordance with any one of the claims 23 to 25, **characterized in that** the implant core (19) is provided on at least one outer side with an introduction passage (55) for the spigot (51) of the implant plate (15, 17) extending from the rim to the cut-out (53).

27. An operation system in accordance with any one of the preceding claims, **characterized in that** at least one implant plate (15, 17) is provided on its inner side with an introduction passage (57) for the implant core (19) extending from the rim to the recess (45).

## Revendications

1. Système chirurgical pour la mise en place d'implants de disques intervertébraux, comprenant :
- au moins un jeu de plaques de travail (11, 13) susceptibles d'être introduites dans un logement de disque intervertébral entre deux corps vertébraux voisins, pour préparer les surfaces des corps vertébraux tournés l'une vers l'autre,
- au moins un implant de disque intervertébral qui comprend deux plaques d'implants (15, 17) appliquées, à l'état implanté, contre les surfaces préparées des corps vertébraux et un noyau d'implant susceptible d'être introduit entre les plaques d'implants (15, 17), et
- un dispositif d'écartement pour pousser en écartement les plaques de travail (11, 3) et les plaques d'implants (15, 17),
et dans lequel le dispositif d'écartement comprend au moins un sabot d'écartement (21) avec au moins un ballon (23) extensible par remplissage avec un fluide et qui est relié à au moins un élément adaptateur (25) qui peut être accouplé à la fois à l'une aux moins des plaques de travail (11, 13) et à l'une au moins des plaques d'implants (15, 7).

2. Système chirurgical selon la revendication 1, **caractérisé en ce que** le sabot d'écartement (21) est susceptible d'être enfilé en coulissement sur la plaque de travail (11, 3) et sur les plaques d'implants (17, 9) au moyen de l'élément adaptateur (25), de préférence sensiblement perpendiculairement à la direction d'écartement.

3. Système chirurgical selon la revendication 1 ou 2, **caractérisé en ce que** la plaque de travail (11, 3) et la plaque d'implant (15, 17) sont chacune pourvue d'au moins un évidement (20) pour recevoir l'élément adaptateur (25).

4. Système chirurgical selon la revendication 3, **caractérisé en ce que** les évidements (20) pour la fixation de l'élément adaptateur (25) dans la direction d'écartement sont pourvus chacun d'une contre-dépouille, en particulier en forme de queue d'aronde.

5. Système chirurgical selon l'une des revendications précédentes, **caractérisé en ce que** l'élément adaptateur (25) est pourvu d'un renfoncement (27) pour recevoir l'enveloppe de ballon non remplie (23).

6. Système chirurgical selon l'une des revendications précédentes, **caractérisé en ce que** le ballon (23) est fermement relié à l'élément adaptateur (25), en particulier par collage.

7. Système chirurgical selon l'une des revendications précédentes, **caractérisé en ce que** l'une au moins des plaques de travail (11, 13) est réalisée en forme de fourche.

8. Système chirurgical selon la revendication 7, **caractérisé en ce que** le dispositif d'écartement comprend au moins deux sabots d'écartement (21), et chaque bras de fourche (29) de la plaque de travail (11) est susceptible d'être accouplé à un sabot d'écartement (21).

9. Système chirurgical selon l'une des revendications précédentes, **caractérisé en ce que** les plaques de travail (11, 13) peuvent être reliées l'une à l'autre au moyen d'au moins un élément de guidage (31), en particulier en forme de tige, et peuvent être réglées en hauteur dans la direction d'écartement l'une par rapport à l'autre le long de l'élément de guidage (31).

10. Système chirurgical selon la revendication 9, **caractérisé en ce qu'**au moins un élément de guidage (31) s'étend, dans la direction d'enfilage, derrière le sabot d'écartement (21) enfilé sur la plaque de travail (11).

11. Système chirurgical selon la revendication 9 au 10, **caractérisé en ce qu'**au moins un élément de guidage (31) s'étend en décalage latéral vers l'extérieur par rapport au sabot d'écartement (21), en particulier dans une zone de coin de la plaque de travail (11, 13).

12. Système chirurgical selon l'une des revendications précédentes, **caractérisé en ce qu'**au moins une plaque de travail (11, 13) présente à une extrémité une jupe (33), placée sous un angle, en particulier de 90°.

13. Système chirurgical selon l'une des revendications précédentes, **caractérisé en ce que** les plaques de travail (11, 13) sont réalisées sous forme de gabarit d'usinage, et sont en particulier pourvues de moyens de guidage (35, 37) pour des outils d'usinage (35), de préférence pour des batteuses à panne et des fraiseuses à mandrin.

14. Système chirurgical selon l'une des revendications précédentes, **caractérisé en ce que** les plaques d'implants (15, 17) présentent chacune sur leur face extérieure un agrandissement (41) en forme de coupole, en particulier sous la forme d'un segment sphérique.

15. Système chirurgical selon l'une des revendications précédentes, **caractérisé en ce que** les faces extérieures des plaques d'implants (15, 17) sont chacune bombée vers l'extérieur, et les bombements (63) sont de préférence prévus en supplément aux agrandissements (41) en forme de coupole.

16. Système chirurgical selon l'une des revendications précédentes, **caractérisé en ce que** les faces extérieures des plaques d'implants (15, 17) présentent chacune une région de bordure plane (65) qui s'étend au moins sur une partie de la périphérie des plaques d'implants (15,17).

17. Système chirurgical selon l'une des revendications précédentes, **caractérisé en ce que** les plaques d'implants (15, 17) présentent chacune sur sa face extérieure au moins une saillie de guidage (43) réalisée en particulier sous forme de panne.

18. Système chirurgical selon l'une des revendications précédentes, **caractérisé en ce que** les plaques d'implants (15,17) sont pourvues chacune sur sa face extérieure d'au moins un évidement (20) pour recevoir l'élément adaptateur (25) du sabot d'écartement (21).

19. Système chirurgical selon l'une des revendications précédentes, **caractérisé en ce que** les évidements (20) se trouvent à l'opposé d'une saillie de guidage (43) réalisée en particulier sous forme de panne.

20. Système chirurgical selon l'une des revendications précédentes, **caractérisé en ce que** les plaques d'implants (15, 17) présente chacune sur sa face intérieure un renfoncement (45) pour recevoir le noyau d'implant (19), et les surfaces d'articulation coopérantes (47, 49) du renfoncement (45) et du noyau d'implant (19) sont de préférence respectivement des surfaces partiellement sphériques.

21. Système chirurgical selon l'une des revendications précédentes, **caractérisé en ce que** le noyau d'implant (19) présente une forme de base à la manière d'une lentille.

22. Système chirurgical selon l'une des revendications précédentes, **caractérisé en ce que** le noyau d'implant (19) présente au moins approximativement la forme de deux segments sphériques appliqués l'un contre l'autre par leurs faces planes, et le centre (11) de l'un des segments sphériques est à l'intérieur de l'autre segment sphérique respectif.

23. Système chirurgical selon l'une des revendications précédentes, **caractérisé en ce qu'**au moins une plaque d'implant (15, 17) présente un tenon (51) en dépassement depuis sa face intérieure et qui, lorsque l'implant est assemblé, pénètre dans un évidement (53) ménagé sur la face extérieure du noyau d'implant (19), et dans lequel l'évidement (53) a une dimension supérieure au tenon (51) pour rendre possible un mouvement relatif entre la plaque d'implant (15, 17) et le noyau d'implant (19).

24. Système chirurgical selon la revendication 23, **caractérisé en ce que** le tenon (51) est agencé au milieu par référence à la dimension de la plaque d'implant en direction sagittale.

25. Système chirurgical selon la revendication 23, **caractérisé en ce que** le tenon (51) est agencé de façon excentrique par référence à la dimension de la plaque d'implant en direction sagittale.

26. Système chirurgical selon l'une des revendications 23 à 25, **caractérisé en ce que** le noyau d'implant (19) est pourvu, sur au moins une face extérieure, d'un canal d'introduction (55) pour le tenon (51) de la plaque d'implant (15, 17), canal qui s'étend de la bordure vers l'évidement (53).

27. Système chirurgical selon l'une des revendications précédentes, **caractérisé en ce qu'**au moins une plaque d'implant (15, 17) est pourvue sur sa face intérieure d'un canal d'introduction (57) pour le noyau d'implant (19), qui s'étend de la bordure vers l'évidement (45).
